# EUROPEAN PATENT APPLICATION

(11) **EP 3 888 701 A1**
(43) Date of publication of application: **06.10.2021**
(21) Application number: 20187976.4
(22) Date of filing: 27.07.2020
(51) Int. Cl.: A61L 2/12, A61L 9/18, A47B 97/00, A47G 27/00, A47K 13/30, B66B 31/00, E03D 9/00, E05B 1/00, H04M 1/17

(54) **AN APPLIANCE FOR VIRUS INACTIVATION**

(30) Priority: 02.04.2020 TR 202005289; 19.05.2020 EP 20175494; 02.06.2020 WO PCT/EP2020/065150; 12.06.2020 EP 20179815
(71) Applicant: SMARTE TEKNOLOJI VE ENERJI SANAYI TICARET A.S., 34467 Istanbul (TR)
(72) Inventor: BALLIKAYA, Melih, 34467 Istanbul (TR)
(74) Representative: Sevinç, Erkan

(57) **Abstract**

The present invention proposes an appliance (100) equipped with an apparatus (1) which includes one or more microwave antenna(s) (11) arranged for emitting a microwave frequency for inciting such mechanical damage in viruses. The microwave antenna(s) (11) can be arranged for emitting a frequency within the range between 1 GHz and 20 GHz; more preferably within the range between 8.0 GHz and 9.5 GHz, even more preferably between 8.2 GHz and 8.8 GHz, even more preferably between 8.3 GHz and 8.5 GHz.

## Description

### Technical field of the invention

The present invention relates to an appliance equipped with an apparatus for inactivating viruses. In particular; the present invention relates to a household appliance arranged for inactivation of viruses using microwave.

### Background of the Invention

Disinfection by inactivation of viruses mainly includes the use of ultraviolet (UV) radiation, or chemical agents such as oxidants.

For inactivation of viruses with the principles of chemistry, chemical agents inevitably contaminate the surfaces to be disinfected, along with any fluid (such as ambient air) in contact with said surfaces. In the cases where the fluid is air or water to be directly contacted with mammals such as humans, the chemical agents should be separated from the fluid for elimination of possible harm. Such separation process brings extra and unmanageable costs. Disinfection by chemical agents take a high amount of time (e.g. around 150 minutes), and cannot be considered to be completed without necessitating such long residence time. Hence, chemical means are not sufficiently suitable for inactivating viruses in a short time. Due to mass transfer limitations, it is practically impossible to achieve a complete disinfection throughout surfaces using chemical agents. Chemical agents used in disinfection from pathogens and viruses include alcohols, oxidizing agents such as hydrogen peroxide and chlorine-based compounds, peracetic acid and aldehydes. Chemical agents can be applied in the form of liquid, aerosol, spray, or vapour. These cannot be safely used in home or office environments, due to their hazardous behaviour when brought into direct contact with humans or foods. Furthermore, upon disinfection process, chemical agents leave remnants (excess and/or by-product chemicals) which also can be considered undesired.

On the other hand, high-energy UV (e.g. UVC) radiation itself is normally insufficient for an effective and swift inactivation of viruses in surroundings of an UV-based disinfection device, unless a tremendous amount of energy is consumed in the process. When air is subjected to UV radiation, ozone is formed, which is harmful to living bodies. Furthermore, when exerted directly, UV radiation is not only harmful to human skin and corneas; but also causes degradation in items such as polymeric materials, pigments/dyestuffs used in colouring the items and surface coatings. In addition, the service life of UVC lamps are short, their energy consumption is high, the UVC rays are only effective when arranged at a correct aspect angle and with a correct light intensity; and does not provide disinfection on sides of surfaces on which UVC rays do not reach. High-energy UVC rays cannot penetrate into items, surface of which are subjected to disinfection process.

WO 2004/069288 A2 discloses the use microwaves as an alternative to UV radiation in sterilization of air. Air is passed through a wire mesh of a sterilization chamber. A vapour or a gas is adhered onto microorganisms to form a complex structure, and then said complex structure is subjected to high energy microwaves or UV radiation to cause a high electrostatic load on the complex structure, resulting in disinfection. Apparently, heating of the complex structure is also an important part of the related disinfection process.

US 2004/ 120 845 A1 relates to removal of pathogens in air circulated by HVAC systems. The system employs an ozone generator for preparing ozone to be mixed with water to obtain an oxidizing chemical agent. UV radiation is used in the formation of ozone from air oxygen. Microwaves are discussed as an alternative to UV radiation. Accordingly, said publication uses microwaves in production of ozone as a step in process for obtaining the oxidizing chemical agent.

It is assumed that in an indoors space, airborne viruses endure for days. In the case where the indoors space is air conditioned, the viruses are easily distributed all around the space, which aggravates a spread. Thus, in the process of normalization after a pandemic such as COVID-19, use of air conditioning systems in public indoor spaces (such as restaurants, workspaces or shopping malls) must be avoided, because air streaming from the air conditioning systems expedites the spread of the infection. Therefore, all of the advantages related to air conditioning shall remain unavailable until the current pandemic is completely over.

Hence, inactivation of viruses in a swift and harmless way is still an unsolved concern.

### Objects of the Invention

The primary object of the present invention is to overcome the drawbacks outlined above.

Another object of the present invention is to propose an appliance to be used in inactivation of viruses in its surroundings.

A further object of the present invention is to propose such appliance which is also easy to produce with low cost, which is easy to operate, which has long service life and easy to maintain by having a simple structure.

A further object according to the present invention is to propose such appliance, which is further arranged for data communication.

### Summary of the Invention

The appliance proposed in the present application has a simple structure, which is easy to produce with low cost. The simple structure is also durable for a long service life, and easy to maintain. The appliance does not consume high amounts of energy during virus inactivation, and is therefore economically advantageous in terms of operational costs. The virus inactivation process does not result in contamination with harmful chemicals such as organic disinfectants or ozone, nor causes health or economic damage.

The appliance according to the present invention includes an apparatus, which comprises microwave antenna arranged for provision of radio frequency to inactivate one or more virus species. The appliance can be further arranged for data transfer, such as that within the context of IoT (i.e. internet of things). The appliance can be further arranged for transmitting such data over the microwave antenna; i.e. by embedding the data onto the waves to be transmitted by the microwave antenna. The appliance can be integrated to a fixture for initiating virus inactivation based on whether the fixture is in use.

In other words, the present invention relates to an appliance comprising an apparatus which includes one or more microwave antenna(s) arranged for emitting a microwave frequency for inciting such mechanical damage in viruses.

Said one or more microwave antenna(s) are preferably arranged for emitting a frequency within the range between 1 GHz and 20 GHz.

Said one or more microwave antenna(s) are more preferably arranged for emitting a frequency within the range between 8.0 GHz and 9.5 GHz, even more preferably between 8.2 GHz and 8.8 GHz, even more preferably between 8.3 GHz and 8.5 GHz.

The apparatus (thus the appliance comprising the same) can be further provided with one or more means for communication.

The means for communication can be arranged for triggering an initiation or an increment of a power consumption of the one or more microwave antenna(s).

The apparatus in the appliance can be arranged for being brought into communication with one or more sensor(s); and can comprise analog/digital connections for the arrangement of the communication between the sensor(s) and the apparatus; and can be pre-programmed to initiate and execute the virus inactivation in accordance with data/signals from the sensor(s).

Throughout the present detailed description, the preposition "in" used in the wording "in the appliance" does not necessarily correspond to the word "inside". Hence the wording "the apparatus in the appliance" is used for stating that the appliance comprises the apparatus. One or more apparatuses can be employed as an inner part (e.g. arranged for emitting microwaves into an inner cavity/compartment etc. of the appliance), and/or as an outer part (e.g. arranged for emitting microwaves to the surroundings of the appliance, e.g. by being provided at an outer surface/shell of the appliance).

In an exemplary embodiment of the appliance, the apparatus can be integrated as a part of a receptacle (e.g. a compartment which can be in the form of a box), such that the one or more microwave antenna(s) are arranged for virus inactivation directed to an inner cavity of the box. Taking into account that several types of household appliances include receptacles (such as compartments or drawers in a refrigerator, or such as a tumbler of a washing and/or drying machine, or such as an inner cavity of a dishwasher, or such as a baking compartment of an oven), one or more inner cavities or compartments of the appliance can be considered as a receptacle or box within the present context. In a further, exemplary embodiment of the appliance, the apparatus can be integrated as a part of an appliance (small home appliance or white good), wherein the one or more microwave antenna(s) are preferably in the form of an outer surface (e.g. shell or top surface) of said appliance.

In an embodiment of the appliance, the apparatus which is described above as being arranged for being brought into communication with one or more sensor(s); and which can comprise analog/digital connections for the arrangement of the communication between the sensor(s) and the apparatus; and which can be pre-programmed to initiate and execute the virus inactivation in accordance with data/signals from the sensor(s):
- In the appliance, said one or more sensor(s) can include one or more load cells (e.g. as pressure sensor(s)) for detecting a change in values of weight exerted onto a top/support surface of the appliance when the appliance is in use, and/or a temperature sensor for detecting thermal values on the top surface. More preferably, the apparatus can be arranged to generate and transfer data related to information based on one or more of said values to a coordinating means for coordination of virus inactivation in a facility where the appliance is placed e.g. via a mesh network or via broadcasting, by embedding the data onto microwaves generated by the microwave antenna(s).

### Brief Description of the Drawings

The appended drawings, brief description of which is provided below, are given solely for the purpose of exemplifying embodiments according to the present invention.

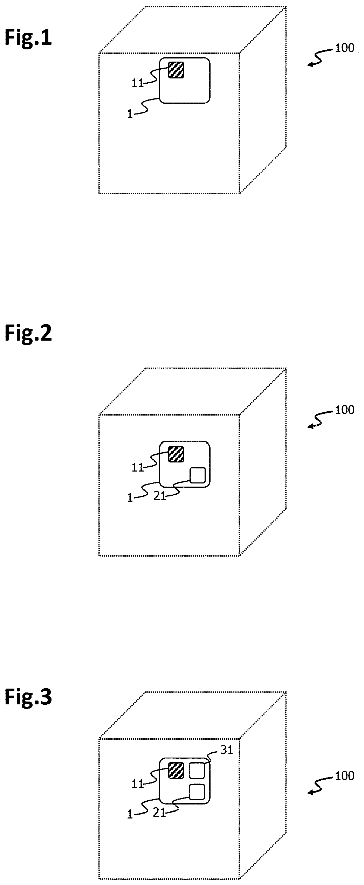

Fig.1 shows a schematic perspective view of an appliance according to the present invention.
Fig.2 shows a schematic perspective view of an exemplary appliance according to the present invention, further provided with a means for communication.
Fig.3 shows a schematic perspective view of an exemplary appliance according to the present invention, further provided with a sensor.

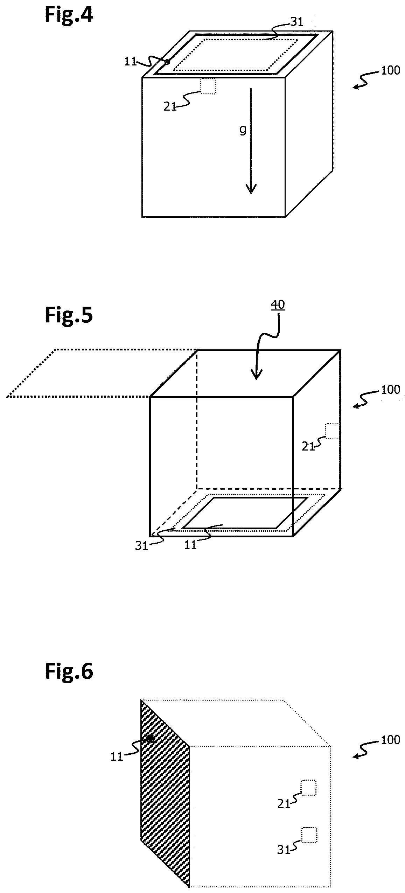

Fig.4 shows a schematic perspective view of an exemplary appliance according to the present invention, wherein the apparatus is integrated to a supporting means ("support surface", which can be an "upper" or "top" surface). In order to clarify the term "top", the gravity vector when the appliance is in use, is represented with "g".
Fig.5 shows a schematic perspective view of an exemplary appliance according to the present invention, wherein an apparatus is integrated to a receptacle, box, compartment (here: single compartment) or inner cavity of the appliance. One or more apparatuses can be placed at a support surface of the receptacle/compartment/box as shown in Fig.5, and/or at an inner side wall or on top of the receptacle/compartment/box such that the inner cavity of the same can be subjected to microwaves. In an aspect within the context of the present application; the receptacle, box or compartment can be regarded as an embodiment of an appliance itself.
Fig.6 shows an exemplary depiction of an appliance (small appliance or white good provided with the apparatus), wherein one or more outer surfaces (here: a side surface) is arranged to function as a microwave antenna to broadcast virus inactivating microwaves to the surroundings of the appliance.

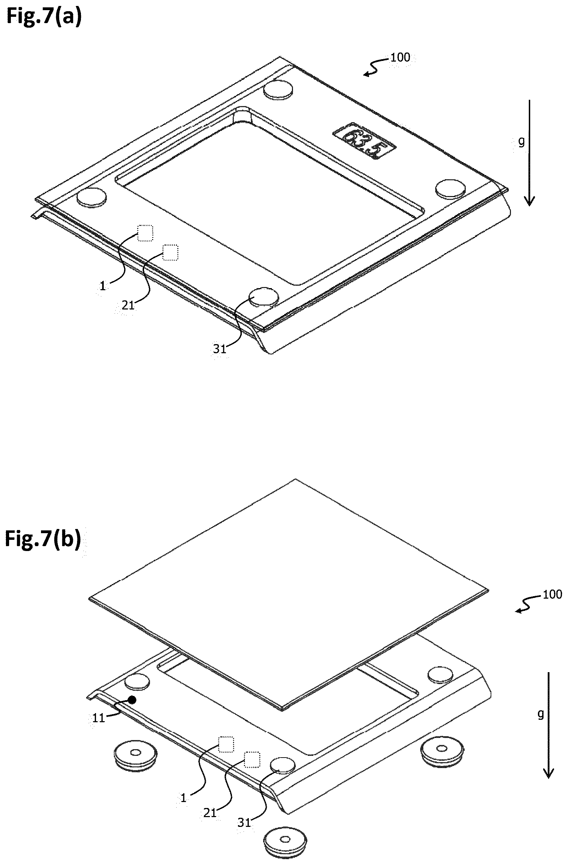

Fig.7(a) shows a perspective view of an exemplary appliance according to the present invention, wherein the appliance is a small home appliance, exemplified as a bascule. Here, an "upper" side of the appliance is equipped with the apparatus (e.g. a top shell of the appliance is arranged as the microwave antenna), thereby enabling the broadcasting of microwaves "upwards" (i.e. in a direction opposite to that of the gravity vector) when the appliance is in use. Fig.7(b) is an exploded view of the appliance shown in Fig.7(a).

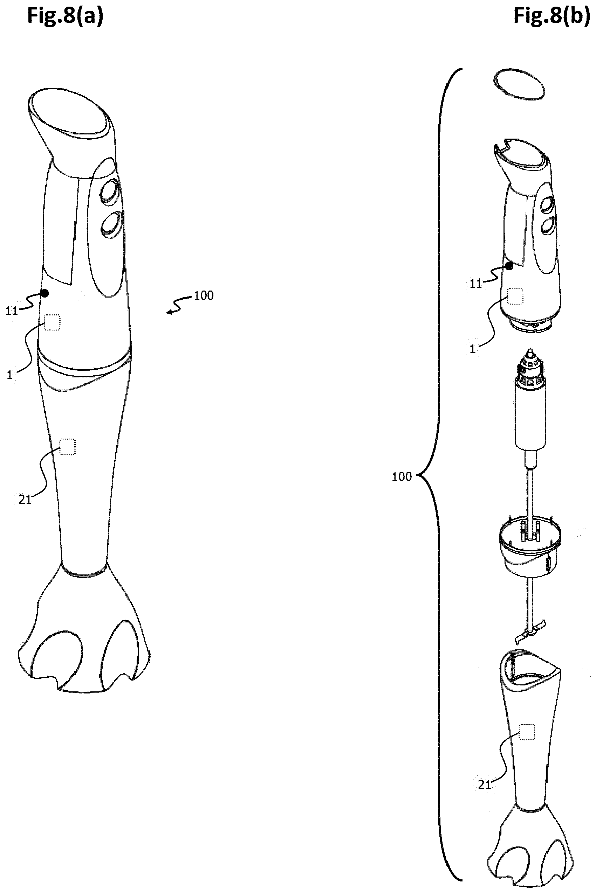

Fig.8(a) shows a perspective view of a handheld blender, as an example to a small home appliance within the context according to the present invention. Fig.8(b) is an exploded view of the appliance shown in Fig.8(a). Here, an outer shell of the appliance is equipped with the apparatus (e.g. a shell of the appliance is arranged as the microwave antenna), thereby enabling the broadcasting of microwaves to the surroundings of the appliance.

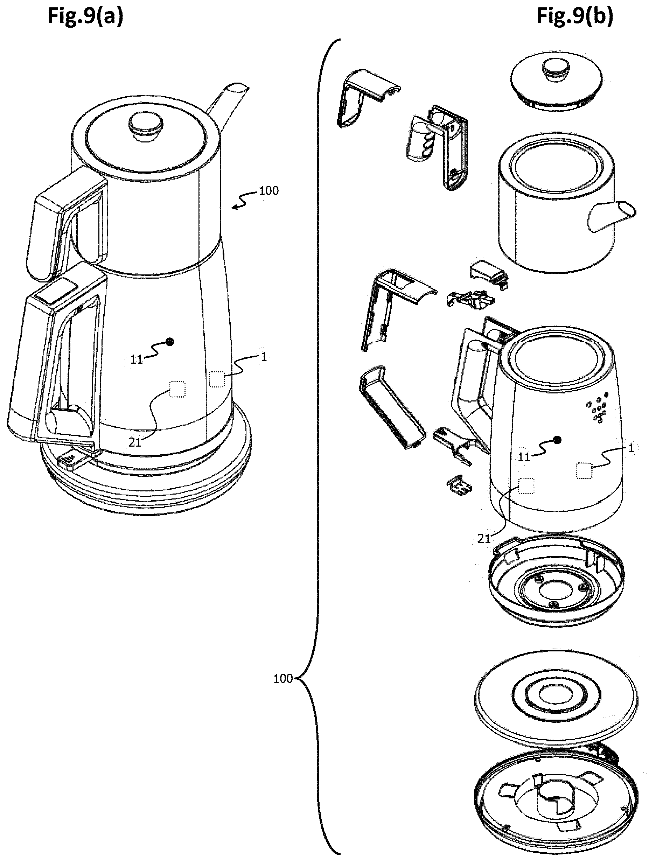

Fig.9(a) shows a perspective view of a tea brewing machine, as an example to a small home appliance within the context according to the present invention. Fig.9(b) is an exploded view of the appliance shown in Fig.9(a). Here, an outer shell of the appliance is equipped with the apparatus (e.g. a shell of the appliance is arranged as the microwave antenna), thereby enabling the broadcasting of microwaves to the surroundings of the appliance.

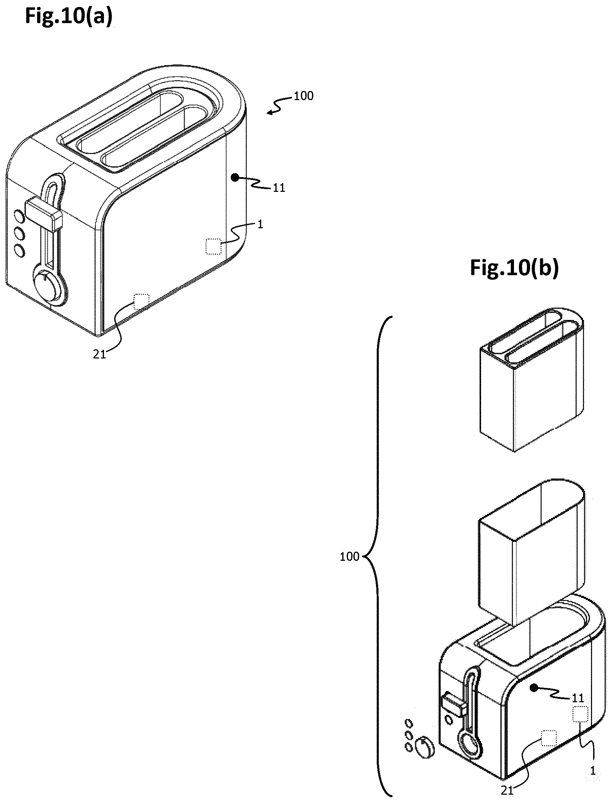

Fig.10(a) shows a perspective view of a toaster, as an example to a small home appliance within the context according to the present invention. Fig.10(b) is an exploded view of the appliance shown in Fig.10(a). Here, an outer shell of the appliance is equipped with the apparatus (e.g. a shell of the appliance is arranged as the microwave antenna), thereby enabling the broadcasting of microwaves to the surroundings of the appliance.

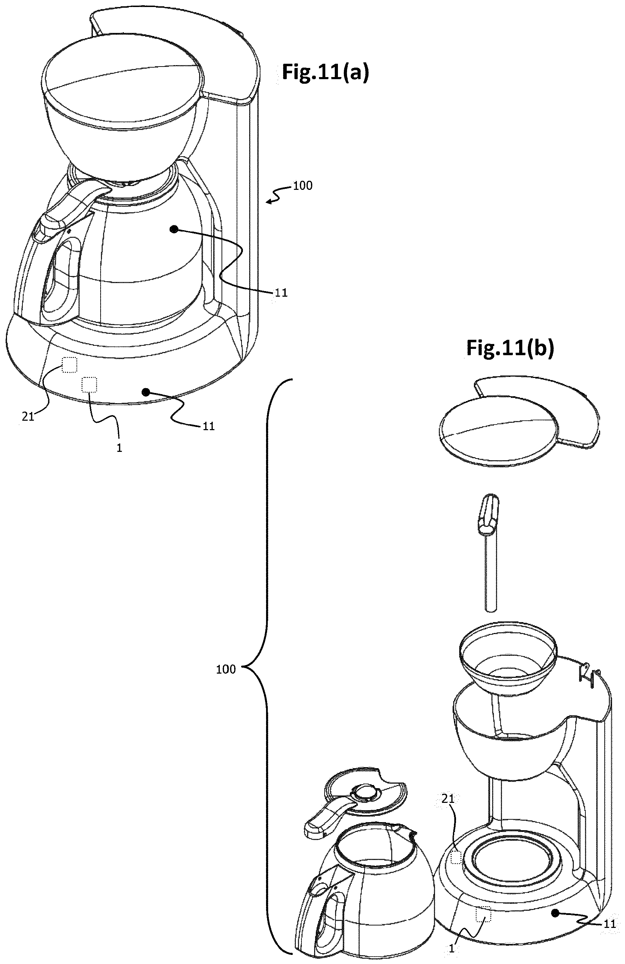

Fig.11(a) shows a perspective view of a coffee brewing machine, as an example to a small home appliance within the context according to the present invention. Fig.11(b) is an exploded view of the appliance shown in Fig.11(a). Here, an outer shell of the appliance is equipped with the apparatus (e.g. a shell of the appliance is arranged as the microwave antenna), thereby enabling the broadcasting of microwaves to the surroundings of the appliance.

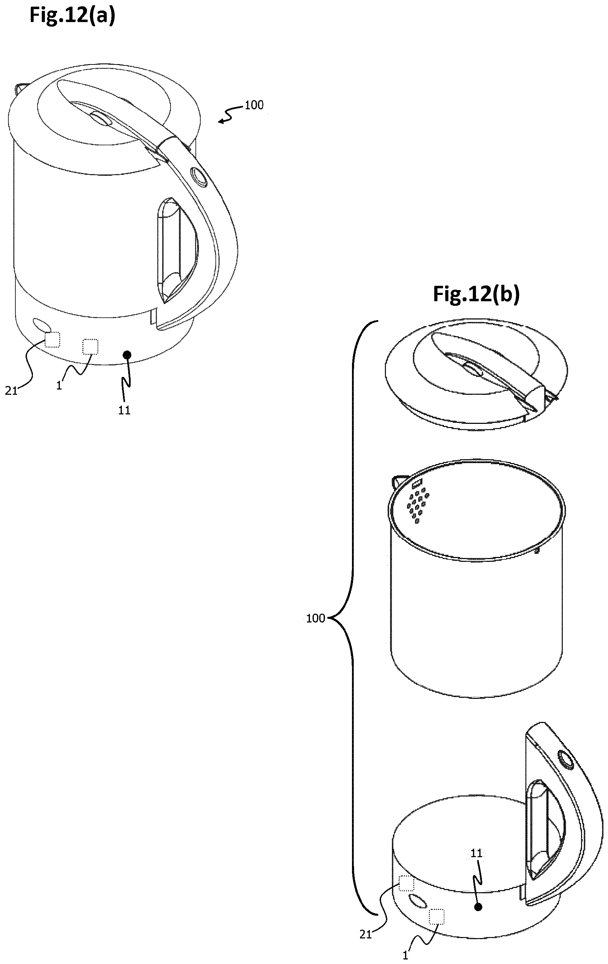

Fig.12(a) shows a perspective view of a kettle, as an example to a small home appliance within the context according to the present invention. Fig.12(b) is an exploded view of the appliance shown in Fig.12(a). Here, an outer shell of the appliance is equipped with the apparatus (e.g. a shell of the appliance is arranged as the microwave antenna), thereby enabling the broadcasting of microwaves to the surroundings of the appliance.

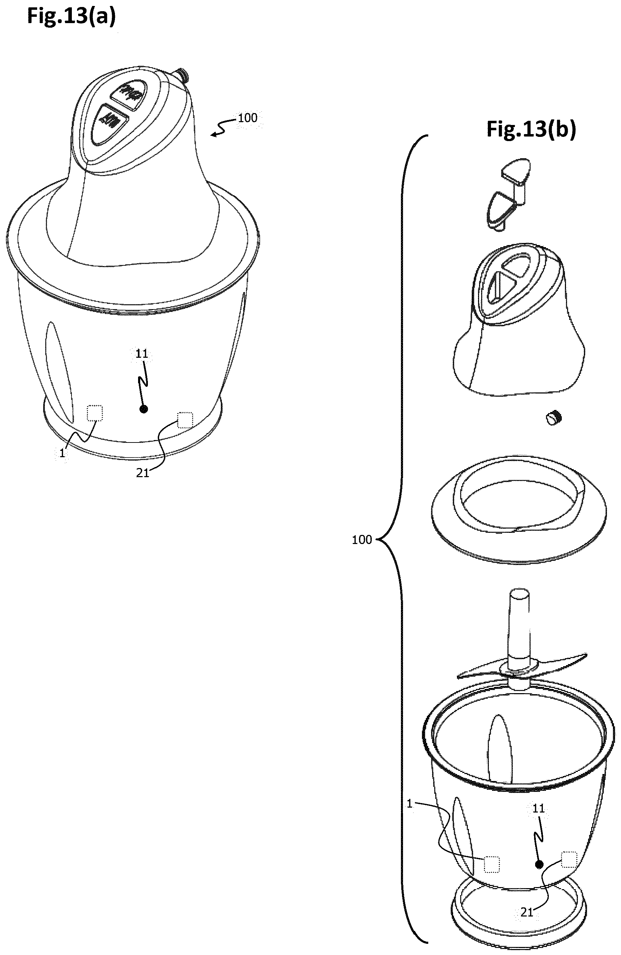

Fig.13(a) shows a perspective view of a food processor, as an example to a small home appliance within the context according to the present invention. Fig.13(b) is an exploded view of the appliance shown in Fig.13(a). Here, an outer shell of the appliance is equipped with the apparatus (e.g. a shell of the appliance is arranged as the microwave antenna), thereby enabling the broadcasting of microwaves to the surroundings of the appliance.

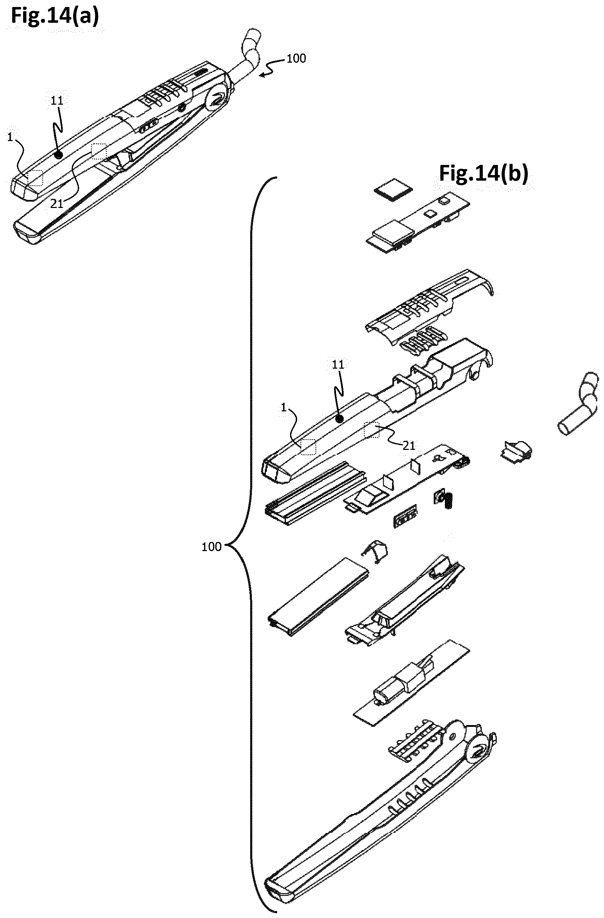

Fig.14(a) shows a perspective view of a hair straightening iron, as an example to a small home appliance within the context according to the present invention. Fig.14(b) is an exploded view of the appliance shown in Fig.14(a). Here, an outer shell of the appliance is equipped with the apparatus (e.g. a shell of the appliance is arranged as the microwave antenna), thereby enabling the broadcasting of microwaves to the surroundings of the appliance.

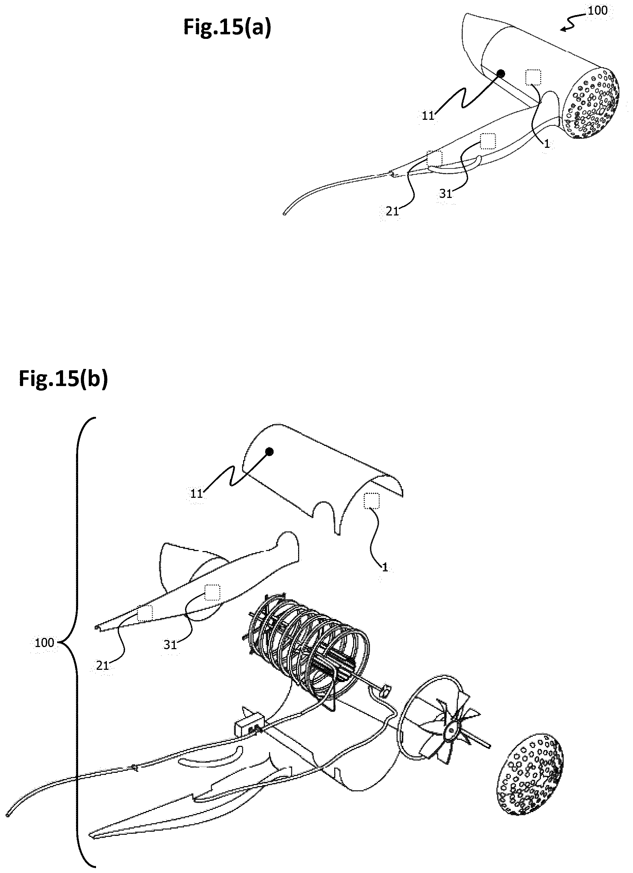

Fig.15(a) shows a perspective view of a hair dryer, as an example to a small home appliance within the context according to the present invention. Fig.15(b) is an exploded view of the appliance shown in Fig.15(a). Here, an outer shell of the appliance is equipped with the apparatus (e.g. a shell of the appliance is arranged as the microwave antenna), thereby enabling the broadcasting of microwaves to the surroundings of the appliance.

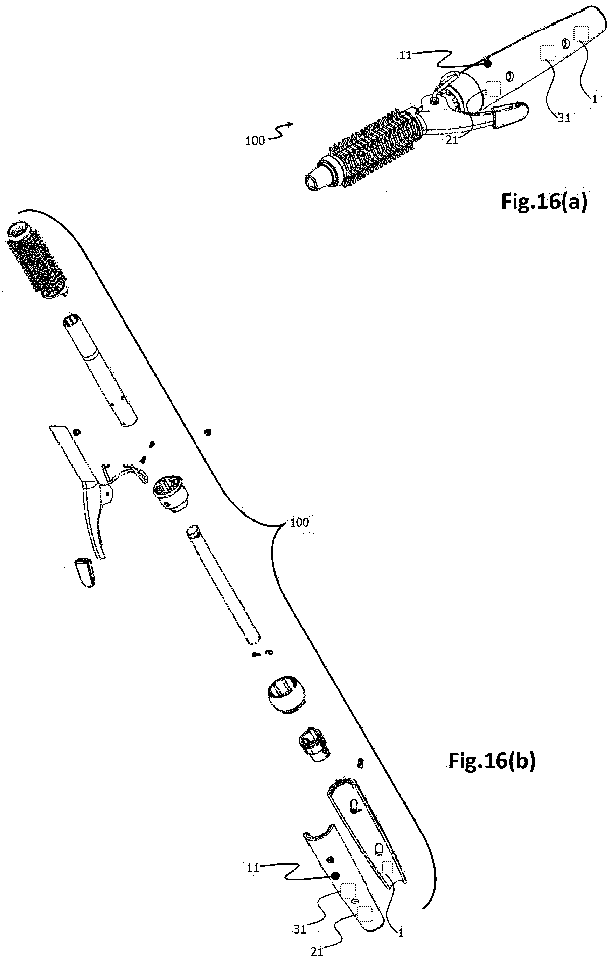

Fig.16(a) shows a perspective view of a hair curling iron, as an example to a small home appliance within the context according to the present invention. Fig.16(b) is an exploded view of the appliance shown in Fig.16(a). Here, an outer shell of the appliance is equipped with the apparatus (e.g. a shell of the appliance is arranged as the microwave antenna), thereby enabling the broadcasting of microwaves to the surroundings of the appliance.

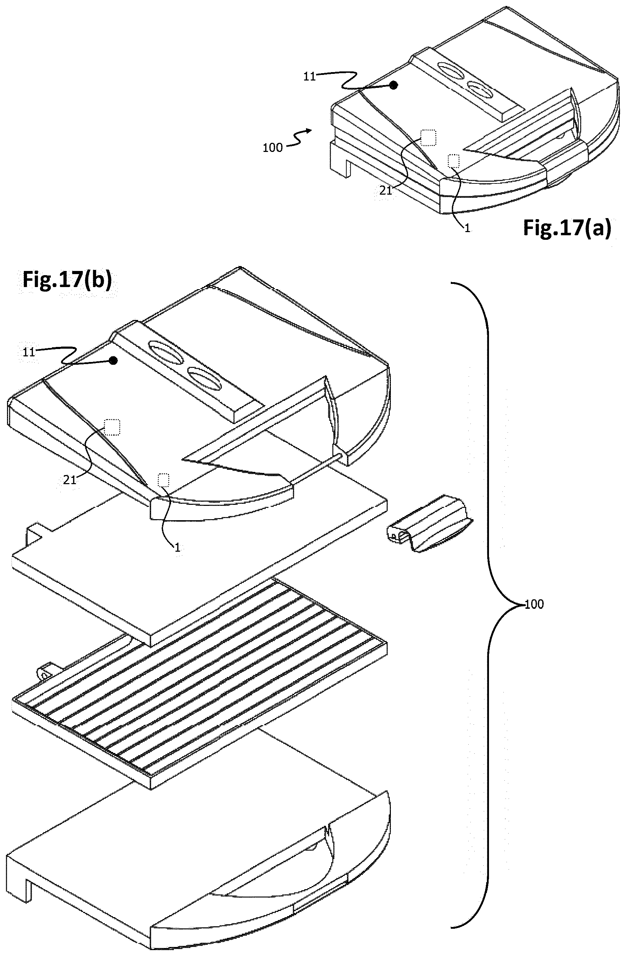

Fig.17(a) shows a perspective view of a sandwich toaster, as an example to a small home appliance within the context according to the present invention. Fig.17(b) is an exploded view of the appliance shown in Fig.17(a). Here, an outer shell of the appliance is equipped with the apparatus (e.g. a shell of the appliance is arranged as the microwave antenna), thereby enabling the broadcasting of microwaves to the surroundings of the appliance.

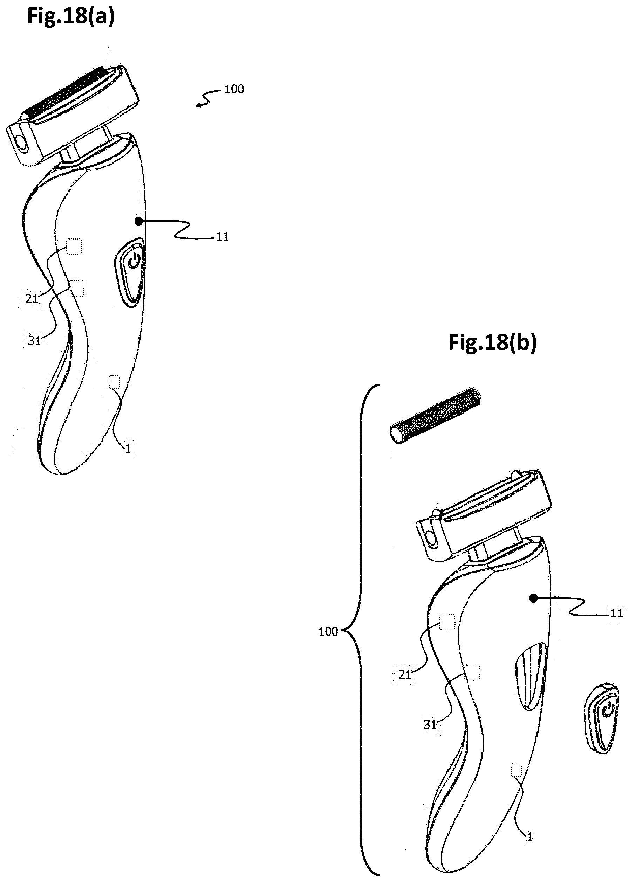

Fig.18(a) shows a perspective view of an electric razor, as an example to a small home appliance within the context according to the present invention. Fig.18(b) is an exploded view of the appliance shown in Fig.18(a). Here, an outer shell of the appliance is equipped with the apparatus (e.g. a shell of the appliance is arranged as the microwave antenna), thereby enabling the broadcasting of microwaves to the surroundings of the appliance.

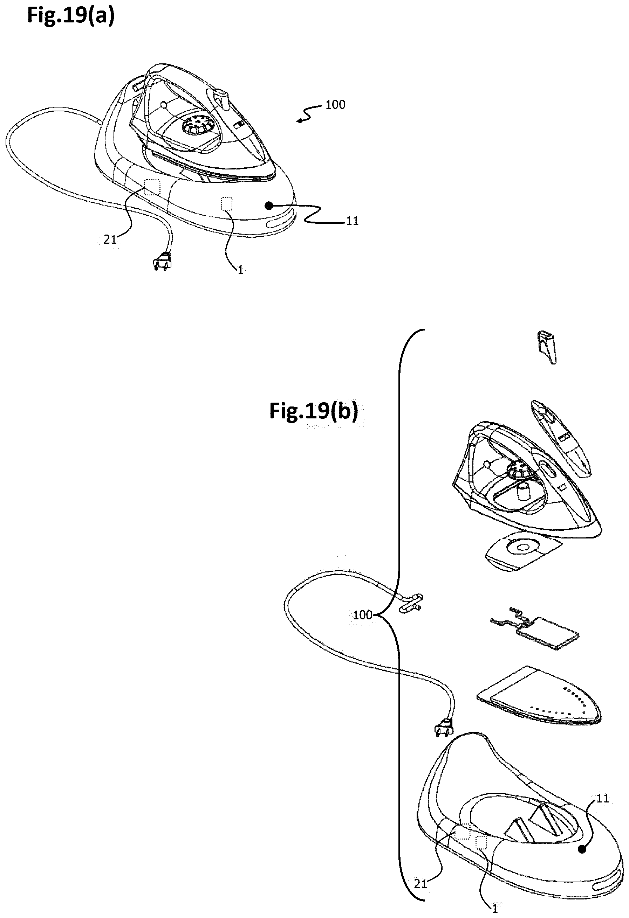

Fig.19(a) shows a perspective view of an iron (here: flat iron), as an example to a small home appliance within the context according to the present invention. Fig.19(b) is an exploded view of the appliance shown in Fig.19(a). Here, an outer shell of the appliance is equipped with the apparatus (e.g. a shell of the appliance is arranged as the microwave antenna), thereby enabling the broadcasting of microwaves to the surroundings of the appliance.

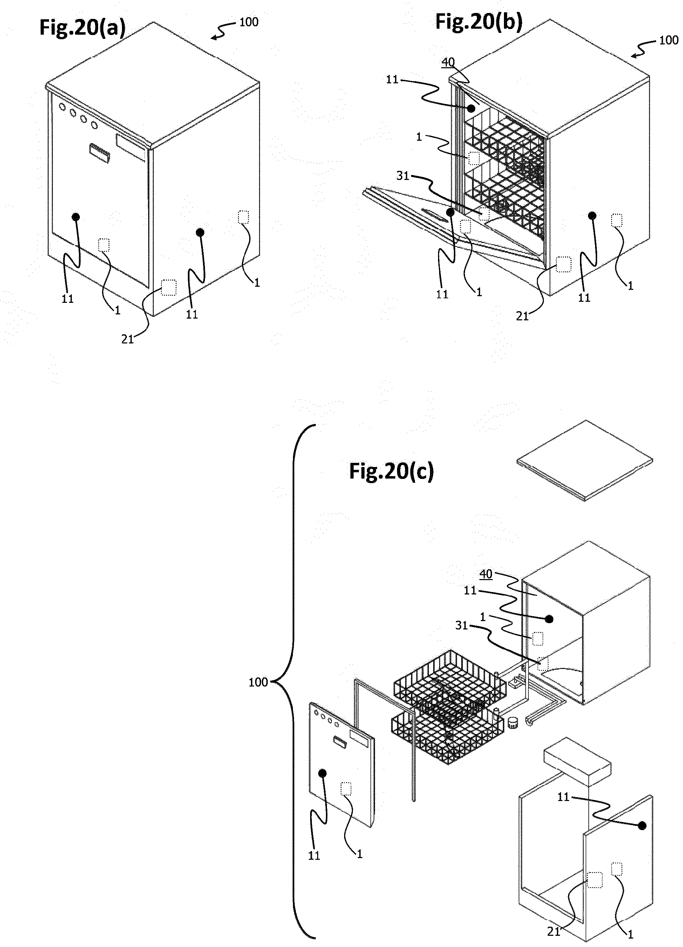

Fig.20(a) shows a perspective view of a dishwasher, as an example to a white good as an appliance within the context according to the present invention. Fig.20(b) shows the same with its lid open, for showing the inner cavity as a receptacle within the context according to the present invention. Fig.20(c) is an exploded view of the appliance shown in Fig.20(a). Here, the appliance is equipped with one or more apparatuses at one or more outer shells for broadcasting of microwaves to the surroundings of the appliance (e.g. a shell of the appliance is arranged as the microwave antenna); furthermore, or alternatively, the appliance is arranged for exertion of microwaves into the inner cavity of the appliance (by means of one or more apparatuses facing the inner cavity), thereby enabling the exertion of microwaves into the inner cavity.

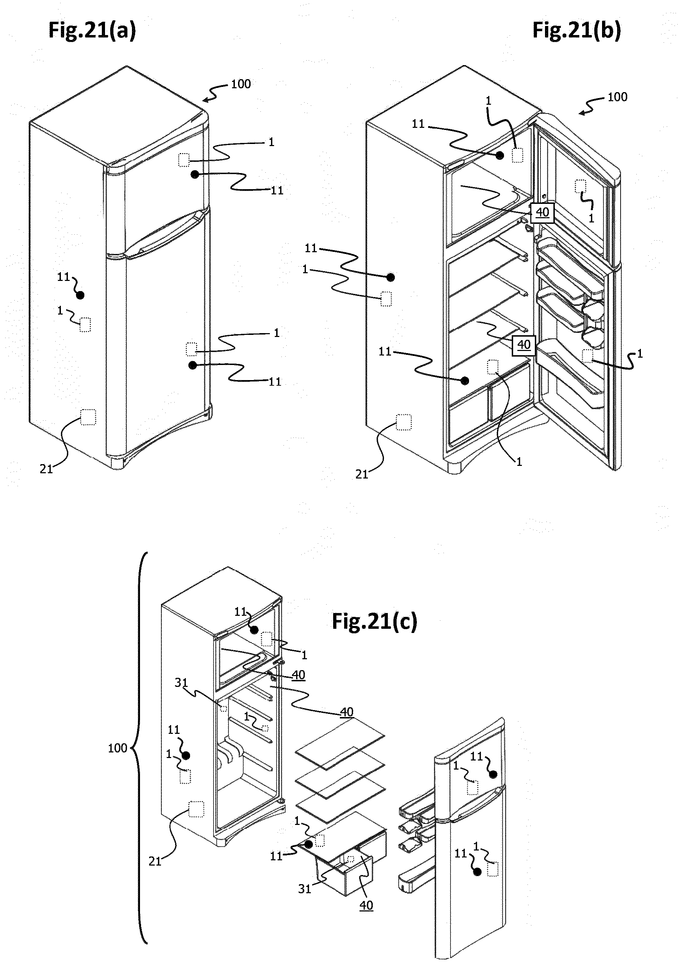

Fig.21(a) shows a perspective view of a refrigerator, as an example to a white good (also applicable to other types of coolers and freezers) as an appliance within the context according to the present invention. Fig.21(b) shows the same with its lid open, for showing the inner cavity as a receptacle within the context according to the present invention. Fig.21(c) is an exploded view of the appliance shown in Fig.21(a). Here, the appliance is equipped with one or more apparatuses at one or more outer shells for broadcasting of microwaves to the surroundings of the appliance (e.g. a shell of the appliance is arranged as the microwave antenna); furthermore, or alternatively, the appliance is arranged for exertion of microwaves into the inner cavity and/or one or more receptacles of the appliance (by means of one or more apparatuses facing (a main) inner cavity; furthermore, or alternatively, by means of one or more apparatuses facing the inner cavity of one or more receptacles (here: drawer)), thereby enabling the exertion of microwaves into the inner cavity.

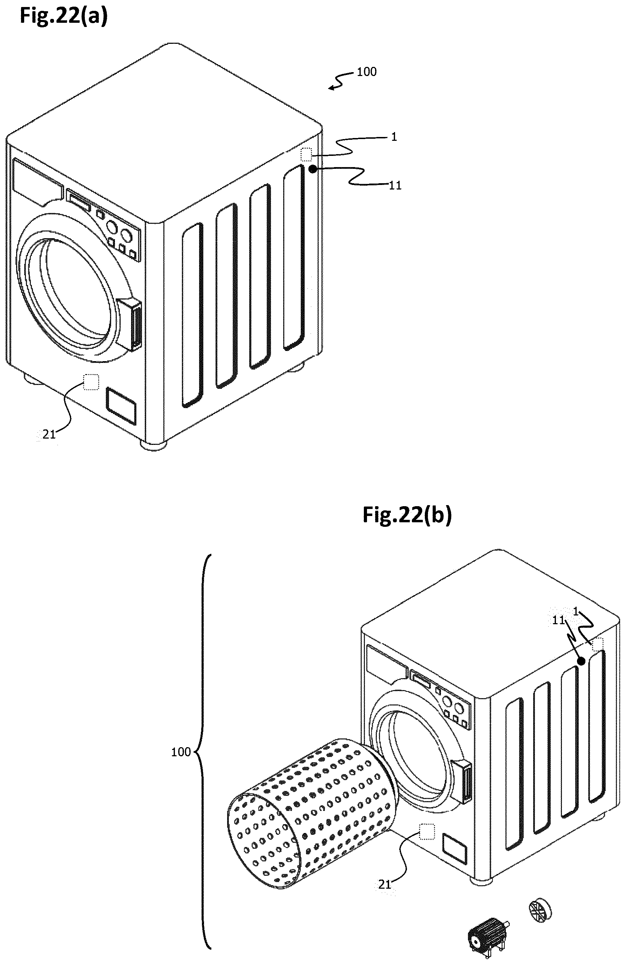

Fig.22(a) shows a perspective view of a washing machine, as an example to a white good (also applicable drying machines and combined washing/drying machines) as an appliance within the context according to the present invention. Fig.22(b) is an exploded view of the appliance shown in Fig.22(a). Here, the appliance is equipped with one or more apparatuses at one or more outer shells for broadcasting of microwaves to the surroundings of the appliance (e.g. a shell of the appliance is arranged as the microwave antenna).

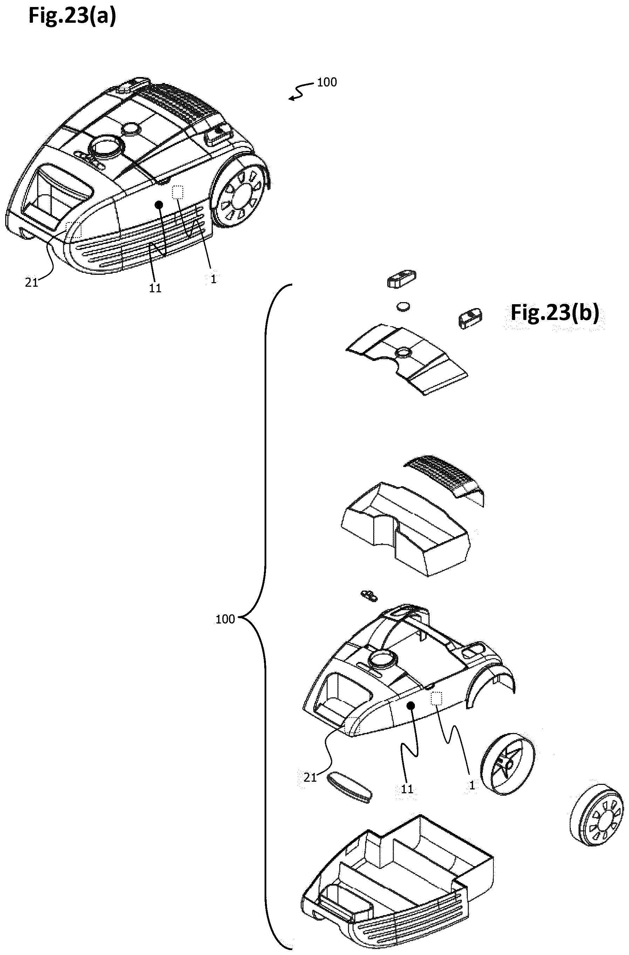

Fig.23(a) shows a perspective view of a vacuum cleaner, as an example to a white good as an appliance within the context according to the present invention. Fig.23(b) is an exploded view of the appliance shown in Fig.23(a). Here, the appliance is equipped with one or more apparatuses at one or more outer shells for broadcasting of microwaves to the surroundings of the appliance (e.g. a shell of the appliance is arranged as the microwave antenna).

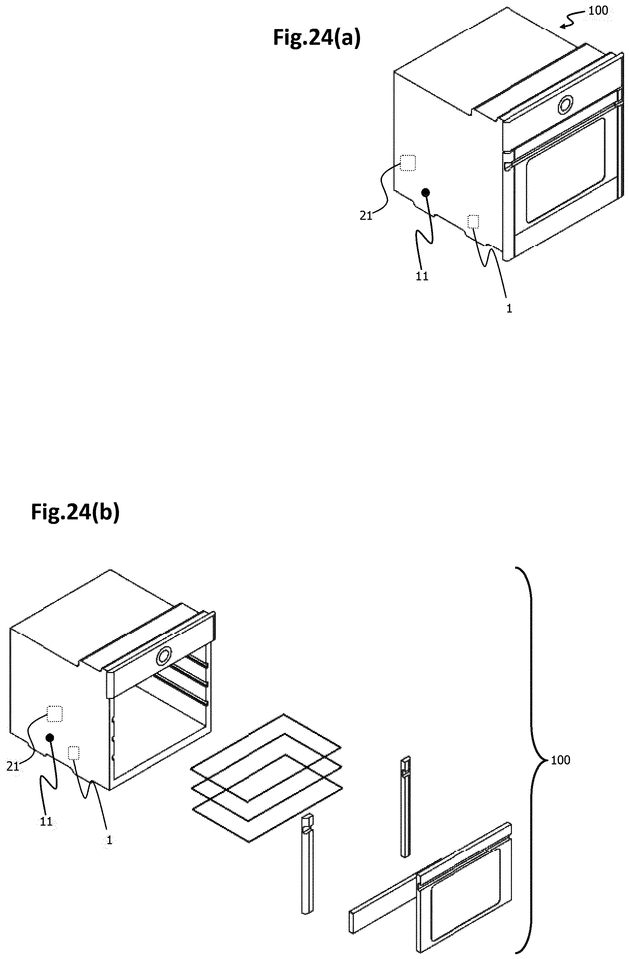

Fig.24(a) shows a perspective view of an oven, as an example to a white good as an appliance within the context according to the present invention. Fig.24(b) is an exploded view of the appliance shown in Fig.24(a). Here, the appliance is equipped with one or more apparatuses at one or more outer shells for broadcasting of microwaves to the surroundings of the appliance (e.g. a shell of the appliance is arranged as the microwave antenna).

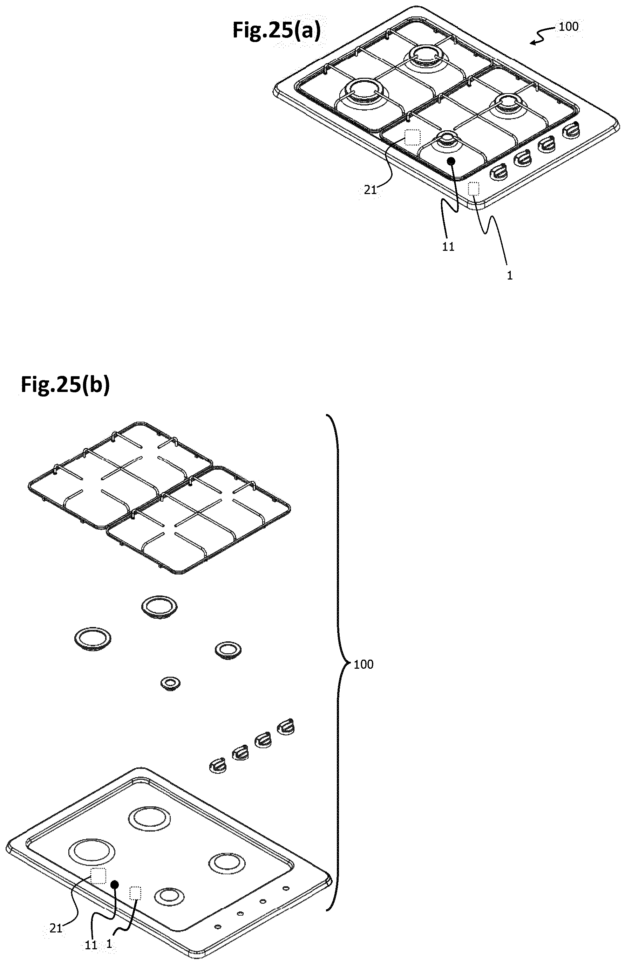

Fig.25(a) shows a perspective view of a cooking range, as an example to a white good as an appliance within the context according to the present invention. Fig.25(b) is an exploded view of the appliance shown in Fig.25(a). Here, the appliance is equipped with one or more apparatuses at one or more outer shells for broadcasting of microwaves to the surroundings of the appliance (e.g. a shell of the appliance is arranged as the microwave antenna).

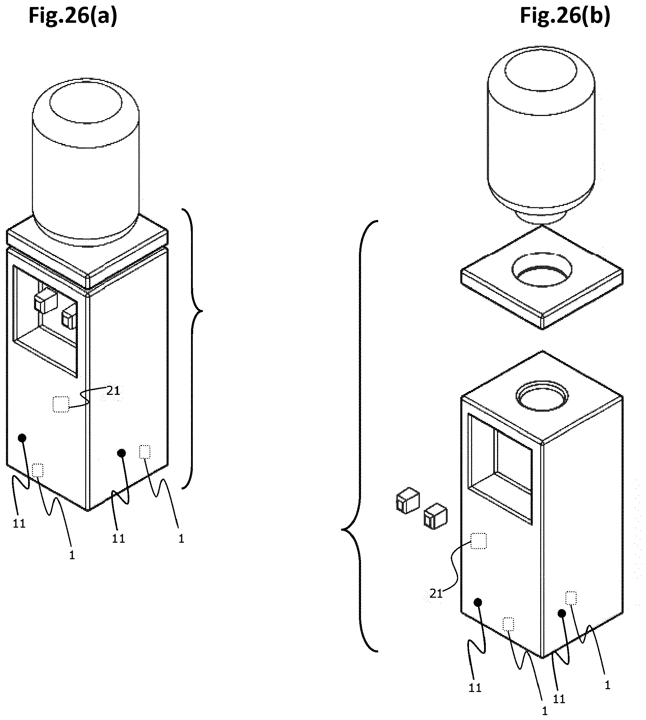

Fig.26(a) shows a perspective view of a water dispenser, as an example to a white good as an appliance within the context according to the present invention. Fig.26(b) is an exploded view of the appliance shown in Fig.26(a). Here, the appliance is equipped with one or more apparatuses at one or more outer shells for broadcasting of microwaves to the surroundings of the appliance (e.g. a shell of the appliance is arranged as the microwave antenna).

### Detailed Description of the Invention

Hereinafter, preferred embodiments of the present invention will be described in detail.

The present specification proposes an appliance (100) which effectively inactivates viruses. The appliance is equipped with an apparatus (1) includes one or more microwave antenna(s) (11) arranged for emitting a microwave frequency for inciting mechanical damage in viruses. Said arrangement can be made using a designated (microwave) frequency for such purpose. In any embodiment of the appliance (100) according to the present invention, the apparatus (1) can be provided with one or more of the following features: programmable analog / digital input outputs, one or more serial communication lines such as SPI (i.e. serial peripheral interface), RS232, RS485, USB, one or more processors, one or more RAM / ROM /FLASH Memories and may act as a SOM (system on module) or SOC (system on chip) or communication module for one or more external MCU(s) (microcontroller units), one or more graphical processor units. Such apparatus (1) (thus the appliance (100) equipped with the same), in particular that arranged for acting as a SOM or communication module for one or more external MCU(s) can be thus considered as a "module". The apparatus (1) (e.g. such "module") (thus the appliance (100) equipped with the same) can include a PCB (printed circuit board), or can be constituted from several other components provided on a PCB. Within the context of the present application, the term "appliance" (100) can be considered to be used instead of "hard good".

The appliance (100) makes use of acoustic vibration, thereby causing resonance which mechanically damages outmost layers of viruses. Although the apparatus (thus the appliance (100) equipped with the same) is effective in inactivation of a wide variety of viruses (such as various types of Ebola, influenza, SARS, etc.) by easily arranging the frequency and energy density levels in accordance with a respective virus; the inactivation of spherical envelops is particularly easy due to their geometric limitations in terms of envelope surface area capable of holding the genetic material and enzymes in their inner volume. Thus, the inactivation occurs instantly (i.e. within a very short residence time) and with consumption of a very low amount of energy. In particular, disintegration of envelopes requires a minimum amount of energy and time, in inactivation of lipid enveloped viruses such as coronaviruses. Yet, advantages of the appliance (100) are not to be limited only to those related to coronaviruses, and it is possible to inactivate other pathogens (viruses and even microorganisms) which require a higher extent of acoustic vibration in disintegration thereof.

Within the context of the present application, the apparatus (1) in the appliance (100) applies acoustic resonance to its surroundings.

Within the context of the present application, the term "microwave" (or "virus inactivating microwave") refers to that with a frequency to induce acoustic vibration on an outermost layer of viruses, causing mechanical damage of such outermost layer. It is observed that virus species can be destroyed in this way, by defining and selecting microwave frequency ranges specific thereto. For instance, it is observed that frequencies within the range between 8.0 GHz and 9.5 GHz cause momentary deformations on envelopes of SARS-COV-2, resulting in rupture of said envelopes. Therefore, this range is considered suitable for being employed in damaging coronaviruses. When the frequency is within the narrower range between 8.2 GHz and 8.8 GHz, the efficiency in inactivation of SARS-COV-2 further increases, and a frequency around 8.4 GHz (i.e. within the range between 8.3 GHz and 8.5 GHz) is considered sweet-spot in inactivation of such viruses in lowest time and with lowest energy consumption.

Since the present invention relies on mechanically damaging viruses by acoustic resonance, the apparatus (1) in the appliance (100) practically does not necessitate nor cause any heating of its surroundings, e.g. fluids or rigid items within an effective electromagnetic field of the apparatus (1) (which can be also named as "range of the one or more microwave antenna(s) (11)"). Considering that heating would denature further biochemical/biological structures other than viruses, the apparatus (1) in the appliance (100) according to the present invention also enables a safe and effective disinfection of useful biological material (e.g. prebiotics and probiotics) from viral activity in a respective medium (e.g. yogurt), within an effective electromagnetic field of the apparatus (1), without damaging said useful biological material. Therefore, the appliance (100) according to the present invention and method of virus inactivation by using such appliance (100) is not to be limited for use in virus inactivation in air, water or rigid surfaces, but also to in-vitro or in-vivo sterilization of other fluids with delicate organic or biological material which should be maintained, such as food products with delicate nutrient ingredients (e.g. above-mentioned useful biological material).

In the appliance (100) according to the present application, the apparatus (1) includes one or more microwave antenna(s) (11) arranged for emitting a microwave frequency for inciting the mechanical damage in viruses by acoustic resonance. Said arrangement can be made by selecting and using a designated (microwave) frequency for such purpose.

The microwave with such designated frequency can be thus applied via said one or more microwave antenna(s) (11), thereby causing acoustic resonance on the outermost layer of viruses. For instance, in SARS-COV-2, the momentary envelope deformations acoustic resonance causes deviations from its original diameter (to extents calculable to be around 30 nm), resulting in inactivation due to mechanical damage in envelope structure. The same applies to other virus species by selection and easily replacing the one or more microwave antenna(s) (11) in accordance with another frequency specific to another virus. Considering different sizes and outmost layer structures, the same principle applies to virus species other than SARS-COV-2, by employing respective specific frequencies selected from a range between 1 GHz and 20 GHz. Considering that currently SARS-COV-2 is the most urgent issue of the world, the frequency is preferably to be kept within the range between 8.0 GHz and 9.5 GHz, more preferably between 8.2 GHz and 8.8 GHz, even more preferably between 8.3 GHz and 8.5 GHz. Considering that commercially available and low cost waveguides (such as WR90) can be used as microwave emitting connectors (11), the frequency can be arranged to be within the range between 6.5 GHz and 13 GHz which provides a single-mode provision of adjustable frequency, and within the range between 7 GHz and 12 GHz for an even higher accuracy. In rod-shaped (i.e. substantially cylindrical) viruses, 5.5-7 GHz provides a peak in resonance, and 20-25 GHz, 33-37 GHz and 45-50 GHz are also effective in inactivation of such viruses. On the other hand, a frequency range within 6-10 GHz is considered as an intersection applicable to inactivation of both spherical viruses and rod-shaped viruses. Hence, the apparatus (1) (thus the appliance (100) comprising the same) can be easily adapted in accordance with a specific frequency designated to inactivation of a target virus.

Fig.1 shows a schematic perspective view of an exemplary embodiment of the apparatus (1) in the appliance (100) according to the present invention, provided with an exemplary microwave antenna (11).

The apparatus (1) can further comprise a means for communication (21), and the apparatus (1) can be thus arranged for data exchange or interchange. Fig.2 shows an exemplary depiction of such embodiment. In this embodiment, the apparatus (1) has internet of things (IoT) compatibility. Such apparatus (1) can be considered as multifunctional. The means for communication (21) can be a means for internet connectivity. Such apparatus (1) can be further arranged to inherit any of the currently known further features related to IoT. Inherently, these considerations also apply to the appliance (100) equipped with the apparatus (1).

Preferably, the means for communication (21) can be arranged for triggering initiating or increasing power consumption of the one or more microwave antenna(s) (11). In such embodiment, the virus inactivation can be initiated or controlled using the means for communication (21). The arrangement of such triggering can be easily made using a suitable actuator.

For instance, this initiation can be performed remotely, e.g. using a remote computing device. Thereby, the virus inactivation can be started as a preparation prior to human approach into the vicinity of the appliance; or the virus inactivation can be performed without necessitating an authorized operator to be physically present near the appliance (100).

Preferably, the means for communication (21) and said microwave antenna(s) (11) can be formed as a single entity. In such embodiment, one or more microwave antenna(s) (11) are arranged for transmitting data embedded on the microwaves they generate/radiate. Thus, the means for communication (21) can be arranged to generate data signals embedded into the radiated microwaves used in virus inactivation. The means for communication (21) can be arranged such that said data signals are embedded on microwaves with any frequency range/value (and related characteristics) explicitly disclosed within the present detailed description directed to inactivate viruses. This embodiment of the apparatus (1) in the appliance (100) enables simultaneous performance of data communication and virus inactivation. The electric field achieved by said microwaves for virus inactivation can be received by a device which can receive said microwaves as data to be interpreted in the form of a signal; and communication between the appliance (100) and the device can thus be established along with virus inactivation. The means for communication (21) can also have IoT compatibility, e.g. by including a TCP/IP stack.

In any one of the embodiments mentioned in the present detailed description, the apparatus (1) (thus the appliance (100) equipped with the same) can be arranged to allow increasing/decreasing momentary power consumption of the microwave antenna(s) (11) to boost/expedite virus inactivation when desired.

In any one of the embodiments, the apparatus (1) can be arranged for being brought into communication with one or more sensor(s) (31) such as an optical "presence" sensor e.g. a light dependent resistor (LDR), and/or a mechanical "presence" sensor e.g. a weight sensor (e.g. load cell) or pressure sensor, and/or a thermal "presence" sensor e.g. a temperature sensor; and such apparatus (1) can comprise analog / digital connections (not shown) enabling such communication between the sensor and the apparatus (1); and the apparatus (1) can be pre-programmed to initiate and execute the virus inactivation in accordance with data/signals from the sensor(s) (31). Fig.3 shows an exemplary depiction of this embodiment. Such embodiment enables that the apparatus (1) (thus the appliance (100) equipped with the same) can be autonomous (i.e. no real-time human intervention is required to initiate and execute virus inactivation), thereby the initiation and execution of virus inactivation can be automatized.

In any one of the embodiments, the means for communication (21) can be pre-programmed as a Doppler radar. Such embodiment can be arranged for normally reciprocate signal broadcast with a suitable, external receiver and function as a typical radar (over the amount of time past until signals echo from said receiver), and simultaneously perform virus inactivation.

One or more apparatus(es) (1) can be integrated as a part of an appliance (100) typically used in houses/apartments or offices (household appliance such as a small home appliance or a white good).

For instance, the apparatus (1) can be integrated as a part of a small home appliance (e.g. bascule), preferably along with one or more sensor(s) (31) (e.g. presence sensors as mentioned above). Such embodiment can be arranged for broadcasting an increased extent of microwave energy (by increasing the power consumption of the microwave antenna (11) when the small home appliance is idle (not in use by physical/mechanical contact by a user). For instance, the sensor(s) (31) (presence sensors) can be one or more load cells (e.g. as pressure sensors) to determine whether a body contact (e.g. body weight) is present on the small home appliance (e.g. bascule): the apparatus (1) can be arranged, at such instance of use (by mechanical contact, e.g. at weighing by means of the bascule), to cease or decrease energy consumption in microwave broadcasting. Alternatively, the small home appliance can be arranged for broadcasting an increased extent of microwave energy (by increasing the power consumption of the microwave antenna (11) when the small home appliance is in use. For instance, the presence sensor(s) (31) can be one or more load cells (e.g. as pressure sensors) to determine whether a physical/mechanical contact is present on the small home appliance (e.g. a body weight is exerted onto the bascule): the apparatus (1) can be arranged, at such instance of use (occupation, mechanical/physical contact, e.g. weighing), to initiate or increase energy consumption in microwave broadcasting, thereby providing virus inactivation (e.g. around soles/feet of the user being weighed by means of the bascule).

Such embodiment can be further arranged to initiate or increase energy consumption in microwave broadcasting in accordance with the use state of the small home appliance. Furthermore, the apparatus (1) (thus, as an appliance (100), the small home appliance equipped with the same) can be provided with one or more further sensors for obtaining one or more information when the small home appliance is in use: the information can include for instance weight of the user, body temperature of the user, or humidity of ambient air. The apparatus (1) (thus the small home appliance equipped with the same) can be further arranged for generating an audible and/or visible signal as a notification related to that a virus inactivation session is in progress, and/or related to that a virus inactivation session is completed. In the latter case, a user can be ensured that it is completely safe to use the small home appliance.

The apparatus (1) can be arranged to generate and transfer data related to said information to a further device (e.g. a coordinating means or coordinator device to coordinate virus inactivation, in communication with the small home appliance), via a mesh network (e.g. by including a modem) or via broadcasting by embedding the data onto said microwaves. In such case, it can be considered that the apparatus (1) (thus the bascule equipped with the same) provides an IoT application/compatibility. By arranging the type, size and shape of e.g. load cells (e.g. as pressure sensors) as presence sensor(s) (31), one or more of such apparatuses (1) can be integrated to one or more of small home appliances within the same context (i.e. to initiate or increase power consumption of microwave antenna(s) (11), in accordance with momentary state of the small home appliance(s) in terms of being idle or in use). Since the apparatus (1) can be produced with a minimal extent of weight, operated with a relatively low extent of power consumption, and expandable virus inactivation coverage area (e.g. by momentarily increasing power consumption of the microwave antenna(s) (11)); it is highly suitable for being integrated to small home appliances.

In such applications, the microwaves generated/radiated/broadcast by the appliance (100) can be subjected to e.g. electrical screening and captured into Faraday's cage in order to prevent data signal pollution to other, irrelevant communication systems in a respective place where the appliance (100) is located.

In other words; in a possible application, the apparatus (1) (thus the appliance (100) equipped with the same) can include the microwave antenna integrated to a supporting means, such as a bascule or a supporting surface at an inner cavity or compartment of a white good as an appliance (100) (e.g. refrigerator). The apparatus (1) can be battery powered and/or arranged to be connected to mains power as a standard power source. The apparatus (1) can be arranged for being brought into communication with one or more sensor(s) (31) (not shown) such as an optical "presence" sensor e.g. a light dependent resistor (LDR), and/or a mechanical "presence" sensor e.g. a weight or load cell (e.g. as pressure sensor), and/or a thermal "presence" sensor e.g. a temperature sensor; and analog/digital connections enabling such communication between the sensor and the apparatus (1); and the apparatus (1) can be pre-programmed to initiate and execute the virus inactivation in accordance with data/signals from the sensor(s) (31). Such embodiment enables that the apparatus (1) (thus the appliance (100) equipped with the same) can be autonomous (i.e. no real-time human intervention is required to initiate and execute virus inactivation), thereby the initiation and execution of virus inactivation can be automatized. Fig.4 shows a schematic perspective view of an exemplary appliance according to the present invention, wherein the apparatus is integrated to a supporting surface ("upper" or "top" surface). In order to clarify the term "top", the gravity vector when the appliance is in use, is represented with "g". Fig.7(a) shows a perspective view of an exemplary appliance according to the present invention, wherein the appliance is a small home appliance, exemplified as a bascule. Here, an "upper" side of the appliance is equipped with the apparatus (e.g. a top shell of the appliance is arranged as the microwave antenna), thereby enabling the broadcasting of microwaves "upwards" (i.e. in a direction opposite to that of the gravity vector) when the appliance is in use. Fig.7(b) is an exploded view of the appliance shown in Fig.7(a).

In particular, said one or more sensor(s) (31) can include a load cell (e.g. as pressure sensor), e.g. provided on the top or support surface of the appliance, for detecting a change in values of weight exerted onto the top surface when the appliance is in use. Said one or more sensors can further include a temperature sensor, e.g. provided on the top surface of the furniture, for detecting thermal values on the top surface. The apparatus (1) (thus the appliance (100) equipped with the same) can be arranged to generate and transfer data related to information based on one or more of said values to a further device (e.g. a coordinating means or coordinator device to coordinate virus inactivation in a facility where the appliance (100) is placed, e.g. a kitchen including a white good e.g. cooler, refrigerator, freezer, dishwasher, oven etc. as an appliance (100)), via a mesh network (e.g. by including a modem) or via broadcasting by embedding the data onto said microwaves. In such case, it can be considered that the appliance (100) provides an IoT application/compatibility.

Within the context of the present application, the white good as appliance can include a receptacle/cavity having a support surface provided with one or more microwave antenna(s) (11) or arranged for receiving microwaves emitted from one or more microwave antenna(s) (11). For instance, when the extent of mechanical force (e.g. weight) exerted onto the support surface momentarily changes (e.g. when items such as food are placed onto the support surface), said one or more sensor(s) (31) detect the state of presence of a respective weight of the items (respectively), and then, virus inactivation on the items can be automatically triggered by initiating or increasing the power consumption of the microwave antenna(s) (11). The temperature sensor can also provide information on ambient temperature or momentary support surface temperature. Such appliance (100) can be in communication with a user interface (such as a touch screen) for enabling communication of user options (such as orders to be taken from a customer as a user) to the above-mentioned further device.

In a further or alternative context; the appliance (100) can be arranged for initiating the virus inactivation based on a user command. Preferably, the apparatus(es) (1) can be arranged for automatically initiating or ceasing the virus inactivation based on a presence sensor data: for instance, the virus inactivation can be initiated when it is detected that nobody is present in the covering range of virus inactivating microwaves of the microwave antenna(s) (11); and/or the virus inactivation can be ceased when it is detected that a person or pet approaches to the covering range of virus inactivating microwaves of the microwave antenna(s) (11).

The appliance (100) with an integrated apparatus (1) within the above context can be for use in public or private buildings. The virus inactivating behaviour of the apparatus (1) is especially useful in public buildings, such as commercial coolers (e.g. supermarket type fridges or chillers, commercial laundry machines). This is because the number of different persons in vicinity of the appliance (100) is expected to be very high when considered per hour.

The appliance (100) can include (or be in the form of) a receptacle (40) (which can be considered as a box or mainly in the form of a cavity/compartment/box) provided with the apparatus (1), such that the one or more microwave antenna(s) (1) are arranged for virus inactivation directed to an inner cavity of the receptacle (40) (e.g. into a compartment which is definable as such box). Fig.5 shows an exemplary depiction of such receptacle (40). For instance; goods or bags brought from shopping can be placed into the cavity/compartment/box (as receptacle (40)), and then the virus inactivation can be triggered/initiated based on a user command, or preferably automatically. Such automatic triggering/initiation can be based on detection of that (e.g. a lid of) the cavity/compartment/box as receptacle (40) is closed. Detection of that the cavity/compartment/box (as receptacle (40)) is closed can be based a signal or data generated by one or more of the following sensors: one or more position sensor(s) (31) for detecting that the cavity/compartment/box (as receptacle (40)) (e.g. a lid thereof) is brought into a closed state; one or more light sensor(s) (31) for detecting that the light received into the cavity/compartment/box (as receptacle (40)) decreases; and/or one or more load cells (e.g. as pressure sensor(s)) (31) for detecting weight of goods when placed into the cavity/compartment/box (as receptacle (40)).

Fig.20(a) shows a perspective view of a dishwasher, as an example to a white good as an appliance (100) within the context according to the present application. Fig.20(b) shows the same with its lid open, for showing the inner cavity as a receptacle (40) within the context of the present application. Fig.20(c) is an exploded view of the appliance shown in Fig.20(a). Here, the appliance (100) is equipped with one or more apparatuses (1) at one or more outer shells for broadcasting of microwaves to the surroundings of the appliance (100) (e.g. a shell of the appliance is arranged as the microwave antenna (11)). Furthermore, or alternatively, the appliance (100) can be arranged for exertion of microwaves into the inner cavity of the appliance (100) (by means of one or more apparatuses (1) facing the inner cavity as a receptacle (40)), thereby enabling the exertion of microwaves into the inner cavity.

Fig.21(a) shows a perspective view of a refrigerator, as an example to a white good (also applicable to other types of coolers and freezers) as an appliance (100) within the context of the present application. Fig.21(b) shows the same with its lid open, for showing the inner cavity as a receptacle (40) within the context of the present application. Fig.21(c) is an exploded view of the appliance (100) shown in Fig.21(a). Here, the appliance (100) is equipped with one or more apparatuses (1) at one or more outer shells for broadcasting of microwaves to the surroundings of the appliance (100) (e.g. a shell of the appliance (100) is arranged as the microwave antenna (11)). Furthermore, or alternatively, the appliance (100) can be arranged for exertion of microwaves into the inner cavity and/or one or more other receptacles (40) (as discussed along the present document, e.g. drawer(s)) of the appliance (100) (by means of one or more apparatuses (1) facing (a main) inner cavity (which can also be considered as a receptacle (40)). Furthermore, or alternatively, by means of one or more apparatuses (1) facing the inner cavity of one or more receptacles (40) (e.g. drawer)), thereby enabling the exertion of microwaves thereinto.

Fig.22(a) shows a perspective view of a washing machine, as an example to a white good (also applicable drying machines and combined washing/drying machines) as an appliance (100) within the context of the present application. Fig.22(b) is an exploded view of the appliance (100) shown in Fig.22(a). Here, the appliance (100) is equipped with one or more apparatuses (1) at one or more outer shells for broadcasting of microwaves to the surroundings of the appliance (100) (e.g. a shell of the appliance is arranged as the microwave antenna (11)). The inner cavity of the tumbler (or drum) can also be considered as a receptacle (40) within the context of the present application.

Fig.23(a) shows a perspective view of a vacuum cleaner, as an example to a white good as an appliance (100) within the context of the present application. Fig.23(b) is an exploded view of the appliance (100) shown in Fig.23(a). Here, the appliance (100) is equipped with one or more apparatuses (1) at one or more outer shells for broadcasting of microwaves to the surroundings of the appliance (100) (e.g. a shell of the appliance is arranged as the microwave antenna (11)).

Fig.24(a) shows a perspective view of an oven, as an example to a white good as an appliance (100) within the context of the present application. Fig.24(b) is an exploded view of the appliance (100) shown in Fig.24(a). Here, the appliance (100) is equipped with one or more apparatuses (1) at one or more outer shells for broadcasting of microwaves to the surroundings of the appliance (100) (e.g. a shell of the appliance (100) is arranged as the microwave antenna (11)). The inner cavity (baking volume) of the oven can be considered as a receptacle (40) within the context of the present application. Fig.25(a) shows a perspective view of a cooking range, as an example to a white good as an appliance (100) within the context of the present application. Fig.25(b) is an exploded view of the appliance (100) shown in Fig.25(a). Here, the appliance (100) is equipped with one or more apparatuses (1) at one or more outer shells for broadcasting of microwaves to the surroundings of the appliance (100) (e.g. a shell of the appliance (100) is arranged as the microwave antenna (11)). Fig.26(a) shows a perspective view of a water dispenser, as an example to a white good as an appliance (100) within the context of the present application. Fig.26(b) is an exploded view of the appliance shown in Fig.26(a). Here, the appliance (100) is equipped with one or more apparatuses (1) at one or more outer shells for broadcasting of microwaves to the surroundings of the appliance (100) (e.g. a shell of the appliance (100) is arranged as the microwave antenna (11)).

In a possible application, the apparatus (1) can be integrated to an appliance (100) such as a small home appliance (e.g. toaster, sandwich toaster, coffee machine, tea brewing machine, kettle, food processor, hair dryer, hair curling iron, hair straightening iron, electric razor, flat iron, pressing iron, bascule, air purifier etc.) or a white good (e.g. refrigerator, dishwasher, laundry/washing machine, oven, range, water dispenser, vacuum cleaner, etc.).

Fig.8(a) shows a perspective view of a handheld blender, as an example to a small home appliance within the context according to the present invention. Fig.8(b) is an exploded view of the appliance shown in Fig.8(a). Here, an outer shell of the appliance is equipped with the apparatus (e.g. a shell of the appliance is arranged as the microwave antenna), thereby enabling the broadcasting of microwaves to the surroundings of the appliance.

Fig.9(a) shows a perspective view of a tea brewing machine, as an example to a small home appliance within the context according to the present invention. Fig.9(b) is an exploded view of the appliance shown in Fig.9(a). Here, an outer shell of the appliance is equipped with the apparatus (e.g. a shell of the appliance is arranged as the microwave antenna), thereby enabling the broadcasting of microwaves to the surroundings of the appliance.

Fig.10(a) shows a perspective view of a toaster, as an example to a small home appliance within the context according to the present invention. Fig.10(b) is an exploded view of the appliance shown in Fig.10(a). Here, an outer shell of the appliance is equipped with the apparatus (e.g. a shell of the appliance is arranged as the microwave antenna), thereby enabling the broadcasting of microwaves to the surroundings of the appliance.

Fig.11(a) shows a perspective view of a coffee brewing machine, as an example to a small home appliance within the context according to the present invention. Fig.11(b) is an exploded view of the appliance shown in Fig.11(a). Here, an outer shell of the appliance is equipped with the apparatus (e.g. a shell of the appliance is arranged as the microwave antenna), thereby enabling the broadcasting of microwaves to the surroundings of the appliance.

Fig.12(a) shows a perspective view of a kettle, as an example to a small home appliance within the context according to the present invention. Fig.12(b) is an exploded view of the appliance shown in Fig.12(a). Here, an outer shell of the appliance is equipped with the apparatus (e.g. a shell of the appliance is arranged as the microwave antenna), thereby enabling the broadcasting of microwaves to the surroundings of the appliance.

Fig.13(a) shows a perspective view of a food processor, as an example to a small home appliance within the context according to the present invention. Fig.13(b) is an exploded view of the appliance shown in Fig.13(a). Here, an outer shell of the appliance is equipped with the apparatus (e.g. a shell of the appliance is arranged as the microwave antenna), thereby enabling the broadcasting of microwaves to the surroundings of the appliance. Fig.14(a) shows a perspective view of a hair straightening iron, as an example to a small home appliance within the context according to the present invention. Fig.14(b) is an exploded view of the appliance shown in Fig.14(a). Here, an outer shell of the appliance is equipped with the apparatus (e.g. a shell of the appliance is arranged as the microwave antenna), thereby enabling the broadcasting of microwaves to the surroundings of the appliance. Fig.15(a) shows a perspective view of a hair dryer, as an example to a small home appliance within the context according to the present invention. Fig.15(b) is an exploded view of the appliance shown in Fig.15(a). Here, an outer shell of the appliance is equipped with the apparatus (e.g. a shell of the appliance is arranged as the microwave antenna), thereby enabling the broadcasting of microwaves to the surroundings of the appliance. Fig.16(a) shows a perspective view of a hair curling iron, as an example to a small home appliance within the context according to the present invention. Fig.16(b) is an exploded view of the appliance shown in Fig.16(a). Here, an outer shell of the appliance is equipped with the apparatus (e.g. a shell of the appliance is arranged as the microwave antenna), thereby enabling the broadcasting of microwaves to the surroundings of the appliance. Fig.17(a) shows a perspective view of a sandwich toaster, as an example to a small home appliance within the context according to the present invention. Fig.17(b) is an exploded view of the appliance shown in Fig.17(a). Here, an outer shell of the appliance is equipped with the apparatus (e.g. a shell of the appliance is arranged as the microwave antenna), thereby enabling the broadcasting of microwaves to the surroundings of the appliance. Fig.18(a) shows a perspective view of an electric razor, as an example to a small home appliance within the context according to the present invention. Fig.18(b) is an exploded view of the appliance shown in Fig.18(a). Here, an outer shell of the appliance is equipped with the apparatus (e.g. a shell of the appliance is arranged as the microwave antenna), thereby enabling the broadcasting of microwaves to the surroundings of the appliance. Fig.19(a) shows a perspective view of an iron (here: flat iron), as an example to a small home appliance within the context according to the present invention. Fig.19(b) is an exploded view of the appliance shown in Fig.19(a). Here, an outer shell of the appliance is equipped with the apparatus (e.g. a shell of the appliance is arranged as the microwave antenna), thereby enabling the broadcasting of microwaves to the surroundings of the appliance.

The apparatus (1) in the appliance (100) can be battery powered and/or arranged to be connected to mains power as a standard power source. The apparatus (1) can be arranged for being brought into communication with one or more sensor(s) (31) (not shown) such as an optical "presence" sensor e.g. a light dependent resistor (LDR), and/or a mechanical "presence" sensor e.g. a load cell as a weight or pressure sensor, and/or a thermal "presence" sensor e.g. a temperature sensor; and analog/digital connections enabling such communication between the sensor and the apparatus (1); and the apparatus (1) (thus the appliance (100) equipped with the same) can be pre-programmed to initiate and execute the virus inactivation in accordance with data/signals from the sensor(s) (31). Such embodiment enables that the apparatus (1) (thus the appliance (100) equipped with the same) can be autonomous (i.e. no real-time human intervention is required to initiate and execute virus inactivation), thereby the initiation and execution of virus inactivation can be automatized.

The apparatus (1) can be arranged to generate and transfer data related to information based on one or more of values generated by the one or more sensor(s) (31) to a further device (e.g. a coordinating means or coordinator device to coordinate virus inactivation in a facility where the hard good (200) is placed, e.g. a kitchen or bathroom including the hard good), via a mesh network (e.g. by including a modem) or via broadcasting by embedding the data onto said microwaves. In such case, it can be considered that the apparatus (1) provides an IoT application/compatibility: for instance, any parameter of e.g. a refrigerator can be remotely followed. These considerations inherently apply to an appliance (100) equipped with one or more of such apparatus(es) (1).

The apparatus (1) (thus the appliance (100) equipped with the same) can be arranged for enabling virus inactivation of a room (e.g. kitchen, bathroom, etc.) by providing an expansive covering area (or volume) of virus inactivating microwaves, e.g. size, shape and power consumption rates of one or more microwave antenna(s) can be arranged for covering a volume of at least 27 m³ (e.g. 3m x 3m x 3m, to cover a bathroom or a kitchen) under an extent of electrical field suitable for virus inactivation.

Within this context, the appliance (100) can be a white good or a small home appliance having one or more outer surfaces (e.g. side surfaces, e.g. body shell, or e.g. lid or door wing) provided with the apparatus (1), such that the one or more outer surfaces include one or more microwave antenna(s) (11). In a possible embodiment, the one or more outer surfaces can function as a microwave antenna (11) as a whole. Fig.6 shows an exemplary depiction of a small appliance or white good provided with the apparatus (1), wherein a side surface is arranged to function as a microwave antenna (11) to broadcast virus inactivating microwaves.

The apparatus (1) can be arranged to generate and transfer data related to information based on one or more of values generated by said one or more sensor(s) (31) to a further device (e.g. a coordinating means or coordinator device to coordinate virus inactivation in a facility where the appliance (100) is placed, e.g. a kitchen or bathroom), via a mesh network (e.g. by including a modem) or via broadcasting by embedding the data onto said microwaves. In such case, it can be considered that the appliance (100) provides an IoT application/compatibility: for instance, any parameter of e.g. a refrigerator can be remotely followed.

Electronic appliances (100) such as white goods and small home appliances, which can be either employed in a fixed place or moved in accordance with their functions, provide an important spectrum for application and integration/innovation in making use of mechanical inactivation of pathogens based on resonance.

An appliance (100) according to the present invention achieves inactivation of pathogens by making use of photons broadcasted in microwave band, which can penetrate into any solid, fluid (e.g. gas or liquid) or evacuated volume, in accordance with (momentary) power consumption of the antenna (11) and respective dielectric constant of a medium into which the penetration of microwaves for disinfection is intended.

The appliance (100) according to the present invention achieves disinfection/inactivation of pathogens without harming food items and without harming mammals (e.g. human) health. Using the nature of phonon (photon-to-photon) interactions, photons broadcasted at specific frequencies cause generation of movement by resonating molecular bonds absorbing the energy of said photons; at walls of the pathogens or their organelles. This movement corresponds to a velocity (thus kinetic energy) which causes the generation of acoustic waves. When the energy extent of said acoustic waves exceed a threshold corresponding to wall resistance of a respective pathogen or organelle, said wall gets mechanically damaged and disinfection/inactivation is thus achieved. Since this type of disinfection requires only the damaging of said walls, the disinfection requires only a low extent of energy consumption. That the penetration of disinfecting microwaves is very high when considering the known limitations of UV (e.g. UVC) or chemical agents, an in-depth (or three-dimensional, or complete) disinfection of items inside or around the appliance can be achieved thanks to the proposed technology.

An appliance (100) according to the present invention can further comprise a control panel (e.g. provided with one or more input means (e.g. buttons/keys, mechanical buttons/keys, capacitive buttons/keys, etc.) to control an electronic card for activation of the microwave antenna (11)), a user interface (e.g. visual e.g. LED, graphical and/or audio interface), one or more processors (or processor group).

An appliance (100) (small home appliances or white goods according to the present invention) can be arranged for momentarily triggering an increased extent of disinfection (boosting or expediting the disinfection) by user command (e.g. through a user interface such as that defined above, also possible by voice command) or automatically (based on sensor data; for instance, by detecting the presence of items, and/or by detecting that an inner cavity (e.g. of a receptacle (40)) is closed (e.g. that a door of a refrigerator as an appliance (100) is closed, thus a user cannot be affected from high energy/amplitude/magnitude microwave to be broadcasted into the inner cavity); using known types of sensors such as laser sensor, capacitive sensor, load cell, LDR, infrared sensor and presence sensors as defined before); thereby enabling a faster disinfection at an instance where no mammal (e.g. human, such as a user) is within the reach of such momentary "high energy" microwave broadcasting. Such high energy microwave broadcasting can be considered as corresponding to power densities exceeding 100 W/m² (specified as a threshold by IEEE C95.1). Thus, the appliance can be arranged for increasing and decreasing power consumption in generation of microwaves at disinfection. The appliance (100) can be also or further arranged for bringing the power consumption rate to a "moderate" extent (which can be also named as a momentary "moderate energy" microwave broadcasting), corresponding to power densities from 10 W/m² up to 100 W/m² (specified by IEEE C95.1, as controlled safety range for controlled environment); and simultaneously actively track mammal (e.g. human) presence within the range of microwave antenna(s) (11) using the "presence" sensor(s) (31) discussed above. The appliance (100) can be also or further arranged for bringing the power consumption rate to a "low" extent (which can be also named as a momentary "low energy" microwave broadcasting), corresponding to power densities up to 10 W/m² (specified by IEEE C95.1, as public safety range, for uncontrolled environment). The appliance (100) can be also arranged for manually or automatically shifting between two or more of the high, moderate and low energy microwave broadcasting modes in accordance with mammal (e.g. human) presence within the range of microwave antenna(s) (11), based on sensor (31) data as discussed before, by changing momentary power consumption rate(s) of said one or more microwave antenna(s) (11). These measures are applicable to any appliance within the context of the present invention: i.e. white goods as well as small home appliances.

The appliance (100) can be also arranged for predicting or determining a dielectric constant of a material (e.g. food items, water, etc.) (e.g. using a camera as sensor (31)) within the range of said one or more microwave antenna(s) (11), and accordingly predict or determine or calculate or estimate a period/duration of inactivation. Such appliance can be further arranged to provide information to a user over an interface (such as a visual, and/or audio interface as discussed before) about said period/duration of inactivation. These measures are applicable to any appliance within the context of the present invention: i.e. to white goods as well as small home appliances.

A preferred embodiment of the appliance (100) according to the present invention can include:
- one or more receptacles (40) (drawer, compartment or box which can be further provided with a lid), and one or more microwave antennas (11) arranged to provide of electric field into an inner cavity of a respective receptacle (40) (e.g. one or more antennas (11) arranged at a side wall, and/or on top of the inner cavity, and/or at or below a support surface for supporting goods to be placed into the inner cavity against gravity, when the appliance is in use); the one or more microwave antenna(s) (11) can further be provided with (e.g. motor-guided) microwave directing means to allow setting and/or manipulation and/or alternation of directions of microwaves; and/or
- one or more receptacles (40) in the form of half-open shelves (i.e. the receptacle is not completely closed thus an inner cavity of the receptacle is available without necessitating opening of a lid or moving of the receptacle (40) as a drawer; such as in the case of an egg shelf or a bottle shelf in a refrigerator), and one or more microwave antennas (11) arranged to provide of electric field into an inner cavity of a respective receptacle (40) (e.g. one or more antennas (11) arranged at a side wall, and/or on top of the inner cavity, and/or at or below a support surface for supporting items to be placed into the inner cavity against gravity, when the appliance is in use); the one or more microwave antenna(s) (11) can further be provided with (e.g. motor-guided) microwave directing means to allow setting and/or manipulation and/or alternation of directions of microwaves; and/or
- one or more shelves for supporting (against gravity) items to be placed into an inner cavity of the appliance (e.g. freezer shelves, or cooler shelves in a freezer, refrigerator or cooler); and one or more microwave antennas (11) arranged to provide of electric field into the inner cavity, to apply disinfection in the inner cavity, thereby enabling disinfection of items in said inner cavity and/or on said shelves; the one or more microwave antenna(s) (11) can further be provided with (e.g. motor-guided) microwave directing means to allow setting and/or manipulation and/or alternation of directions of microwaves;
- one or more microwave antennas (11) arranged for broadcasting microwaves for disinfecting surfaces and/or volumes around the appliance, in one of more of the following directions when the appliance is in use: sideways (i.e. substantially orthogonal to gravity vector; preferably the one or more antennas (11) are in the form of an outer shell or side wall of the appliance), and/or upwards (i.e. substantially opposite to the direction of gravity vector; preferably the one or more antennas (11) are in the form of a support surface or an upper shell or an upper wall of the appliance), and/or downwards (i.e. parallel to the gravity vector, opposite to "upwards" defined before);
- one or more electronic cards for controlling (which can include initiation, increasing/decreasing power consumption rate, ceasing of the operation of any of the above mentioned microwave antennas (11).

The one or more sensors (31) can be selected from the list below, for functioning (for being operated) within any of the sensor-related contexts discussed above:
- real-time capacitive sensors,
- capacitive schalt sensors (e.g. in a plurality, to be vertically positioned against a side surface of the pot, thereby each of the capacitive schalt sensors corresponding to different levels of the liquid mixture).
- temperature probes.
- optical sensors (such as cameras).
- IR distance sensors,
- thermophile sensors,
- microwave sensors,
- microwave sensors with LDR flight dependent resistor),
- RGB (red/green/blue) color space distance sensors.
- LDR sensors along with a light source.
- ultrasonic sensors.
- time of flight sensors, or
- combinations thereof.

Thanks to the apparatus (1) comprising the microwave antenna(s) (11), the appliance (100) achieves the inactivation of viruses via acoustic vibration, bv emitting a microwave frequency for inciting material damage in viruses. It can be considered equivalent whether the mechanical damage occurs in outermost layers of viruses, or in genetic material of viruses which remain inside the outermost layers, or any other part of the viruses. Thus, the present application can be considered to be related to appliance (100) provided with one or more apparatus(es) (1) which include one or more microwave antenna(s) (11) arranged for emitting a microwave frequency for inciting mechanical damage in viruses via acoustic vibration.

Furthermore, the modern technology allows the initiation or increment in power radiation by microwave antenna(s) (11) without substantially affecting the power consumption of the microwave antennas. Therefore, within the context of the present application discussed before, "the triggering of initiation or increment of a power consumption of the one or more microwave antenna(s) (11)" can be considered equivalent to "the triggering of initiation or increment of a power radiation by the one or more microwave antenna(s) (11)".

### Reference signs:

- 1: apparatus
- 11: microwave antenna(s)
- 21: means for communication
- 31: sensor
- 40: receptacle
- 100: appliance

## Claims

1. An appliance (100) provided with one or more apparatus(es) (1) which include one or more microwave antenna(s) (11) arranged for emitting a microwave frequency for inciting mechanical damage in viruses via acoustic vibration.

2. The appliance (100) according to the claim 1, wherein said one or more microwave antenna(s) (11) are arranged for emitting a frequency within the range between 1 GHz and 20 GHz.

3. The appliance (100) according to the claim 2, wherein said one or more microwave antenna(s) (11) are arranged for emitting a frequency within the range between 8.0 GHz and 9.5 GHz, more preferably between 8.2 GHz and 8.8 GHz, even more preferably between 8.3 GHz and 8.5 GHz.

4. The appliance (100) according to any of the claims 1 to 3, further comprising one or more means for communication (21).

5. The appliance (100) according to the claim 4, wherein the means for communication (21) is arranged for triggering an initiation or an increment of a power radiation by the one or more microwave antenna(s) (11).

6. The appliance (100) according to any of the claims 1 to 5, wherein the one or more apparatus(es) (1) are arranged for being brought into communication with one or more sensor(s) (31); and the apparatus(es) (1) comprise analog/digital connections for arrangement of the communication between the sensor(s) (31) and the apparatus (1); and the apparatus (1) is pre-programmed to initiate and execute the virus inactivation in accordance with data/signals from the sensor(s) (31).

7. The appliance (100) according to the claim 6, said one or more sensor(s) (31) including one or more load cell(s) for detecting a change in values of force exerted onto a support surface of the appliance (100) when the appliance (100) is in use; and/or a temperature sensor for detecting thermal values on the support surface; more preferably the appliance (100) is arranged to generate and transfer data related to information based on one or more of said values to a coordinating means for coordination of virus inactivation in a facility where the appliance (100) is placed via a mesh network or via broadcasting by embedding the data onto microwaves generated by the microwave antenna(s) (11).

8. The appliance (100) according to any of the claims 1 to 7, wherein the one or more microwave antenna(s) (11) are in the form of an outer shell of the appliance (100).

9. The appliance (100) according to any of the claims 1 to 7, comprising a receptacle (40), wherein the one or more microwave antenna(s) (1) are arranged for virus inactivation directed to an inner cavity of the receptacle (40).

10. The appliance (100) according to any of the claims 1 to 9, wherein the appliance (100) is a white good.

11. The appliance (100) according to the claim 10, wherein the white good is selected from refrigerator, dishwasher, laundry machine and washing machine.

12. The appliance (100) according to the claim 10, wherein the white good is selected from oven, range, water dispenser and vacuum cleaner.

13. The appliance (100) according to any of the claims 1 to 8, wherein the appliance (100) is a small home appliance.

14. The appliance (100) according to any of the claims 1 to 8, wherein the small home appliance is selected from toaster and coffee machine.

15. The appliance (100) according to any of the claims 1 to 8, wherein the small home appliance is selected from sandwich toaster, tea brewing machine, kettle, food processor, hair dryer, hair curling iron, hair straightening iron, electric razor, flat iron, pressing iron, vacuum cleaner, bascule and air purifier.
